# EUROPEAN PATENT APPLICATION

(11) **EP 4 666 978 A2**
(43) Date of publication of application: **24.12.2025**
(21) Application number: 25214851.5
(22) Date of filing: 14.11.2023
(51) Int. Cl.: A61B 34/20

(54) **METHOD AND SYSTEM FOR ANALYSIS AND PRESENTATION OF CONNECTIONS BETWEEN ABLATION POINTS**

(30) Priority: 16.11.2022 US 202217988029
(62) Divisional of application: 23824959.3
(71) Applicant: Biosense Webster (Israel) Ltd., 2066717 Yokneam (IL)
(72) Inventor: ZAIDES, Leonid, 2066717 Yokneam (IL); GOLDBERG, Stanislav, 2066717 Yokneam (IL); ZOUBI, Alaa, 2066717 Yokneam (IL); GOLAN, Ariel, 2066717 Yokneam (IL)
(74) Representative: Carpmaels & Ransford LLP

(57) **Abstract**

A method, apparatus and computer program product, the method comprising: obtaining coordinates of locations in a body part of a patient; determining at least one trajectory comprising at least part of the locations; determining whether a connection between at least two locations connected along the at least one trajectory exceeds a threshold; providing a visual representation of the body part, including at least some of the locations and visual indications of connections between locations; and subject to the connection exceeding the threshold, providing a visual indication of the connection weight.

## Description

### FIELD OF THE DISCLOSURE

This disclosure relates to medical applications in general, and to assessing and representing the connections between ablation points, in particular.

### BACKGROUND OF THE DISCLOSURE

Arrhythmias may be caused by problems with the electrical conduction system of the heart, and in particular electrical activity in one or more points or areas on a wall of a heart chamber. Atrial fibrillation is an arrhythmia characterized by disorganized signals that make the atria (left and/or right atria) squeeze very fast and in an asynchronous cardiac rhythm.

A common treatment of atrial fibrillation, also referred to as A-fib, is ablation which uses energy to create scars on one or more active areas on the heart wall, in order to block faulty electrical signals that contribute to the disorganized signals and to restore typical heartbeat. Ablation can be performed using one or more types of energy to create the scars, such as but not limited to Radio-Frequency (RF), pulsed-field (sometime referred to as PFA or irreversible electroporation (IRE)), focused laser, ultrasound, heat, or the like.

### BRIEF DESCRIPTION OF THE DRAWINGS

The present disclosure will be more fully understood from the following detailed description of the examples thereof, taken together with the drawings, in which:
Fig. 1 is a schematic, pictorial illustration of a catheter-based electrophysiological (EP) mapping and ablation system, in accordance with some exemplary embodiments of the disclosure;
Fig. 2A is a schematic illustration of a heart undergoing ablation, in accordance with some exemplary embodiments of the disclosure;
Fig. 2B is a schematic illustration of a user examining a heart having multiple sites where ablation has been applied, in accordance with some exemplary embodiments of the disclosure;
Figs. 3A and 3B demonstrate the effect of a larger distance in three-dimensional space appearing shorter in a projection over a two-dimensional plane;
Fig. 3C shows the points as in Fig. 3B, with supplemented connecting lines, in accordance with some embodiments of the disclosure;
Fig. 4 is a flowchart of steps in a method for determining and displaying ablation sites and connections therebetween, in accordance with some exemplary embodiments of the disclosure;
Figs. 5A and 5B demonstrate examples of the segments connecting ablation locations, as created by a traditional solution;
Figs. 5C-5F demonstrate examples of the connecting segments created by existing solutions and some exemplary embodiments of the disclosure;
Fig. 6 shows an image of a heart as displayed over a display device, with ablation points, and with estimated trajectories, in accordance with some embodiments of the disclosure; and
Fig. 7 is a schematic block diagram of a computing platform for determining and indicating a connection between ablation points that exceeds a predetermined threshold, in accordance with some exemplary embodiments of the disclosure.

### DETAILED DESCRIPTION OF EXAMPLES

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client-server environment, such software programming code may be stored on a client or a server. The software programming code may be embodied on any of a variety of known media for use with a data processing system. This includes, but is not limited to, magnetic and optical storage devices such as disk drives, magnetic tape, compact discs (CD's), digital video discs (DVD's), and computer instruction signals embodied in a transmission medium with or without a carrier wave upon which the signals are modulated. For example, the transmission medium may include a communications network, such as the Internet. In addition, while the invention may be embodied in computer software, the functions necessary to implement the invention may alternatively be embodied in part or in whole using hardware components such as application-specific integrated circuits or other hardware, or some combination of hardware components and software.

### OVERVIEW

It is known that atrial fibrillation can be triggered by aberrant conduction pathways that originate in muscle bundles that extend from the atrium to the pulmonary veins.

Cardiac ablation is a procedure that locally heats and ablates cardiac tissue on the inner surface of a heart cavity so as to relieve cardiac dysfunction, such as atrial fibrillation. For example, ablation can produce electrical pulmonary vein isolation, thereby regaining sinus rhythm.

As a physician, typically a cardiologist, performs the ablation therapy, the physician typically forms ablation lesions by applying energy to the heart tissue using an ablation electrode positioned at a distal end of a catheter. The ablation electrode contacts tissue in the heart cavity at multiple discrete ablation sites along a predefined trajectory. Depending on the ablation area and the required results, the defined trajectory may be open, i.e., a line with start and end points, or form a closed loop.

The cardiologist may monitor the procedure by observing the position of the catheter tip in an image of the heart on a display. The catheter tip position can be detected, for example, by a catheter position tracking system or imaging system.

If the cardiologist creates adjacent ablation lesions that are too far apart, the resulting gap may not completely eliminate the parasitic electrical pathways of the cardiac activation wave, for example, and the cardiac dysfunction may not be completely alleviated.

During the procedure, for each site at which ablation is applied, the three-dimensional (3D) location and optionally additional features, such as a quality measure of the ablation, the temperature at which the ablation was created, the heart wall thickness, or the like may be stored. The sites may be displayed to the physician during and/or post operation, over a display device showing an image of the heart or part thereof.

Currently, physicians may visually estimate if the ablation locations form the required trajectory (sometimes referred to as a "ablation path" or "ablation line"), whether it is closed or open, and whether the distance between any two consecutive sites along the trajectory is small enough to eliminate parasitic electrical pathways. However, the visual estimation may not be accurate enough. Additionally, projecting the 3D ablation locations on the two-dimensional (2D) display may distort the distances and disable a reliable determination.

Naive methods of following the creation time of the ablation sites, if available, and determining the distance between any two successively created ablations may also not provide full solution, as the physician may revisit certain areas after ablating other areas, to create another ablation between two previously created ones. Additionally, a physician may create ablations in two or three distinct areas, wherein the distance between the areas is large, but this does not pose a problem.

Some computational solutions have been proposed to determine the trajectory and distances, and in particular identifying distances within the same area that exceed a threshold. However, no such solution has proven satisfactory.

Thus, embodiments of the present disclosure provide a system and method for more accurately determining and optionally visualizing the trajectories along which the ablation points are located, and the connections between related cardiac ablation sites. In some embodiments, a processor of a cardiac mapping and ablation system may receive the coordinates of multiple ablation sites on the surface of the heart. The processor may then apply a unique combination of computational methods for determining the connectivity between the ablation sites. The processor may then cause the visualization of some or all of the connections. For example, each connection may be marked with a color, width, pattern or additional indicators representing its size or size range. For example, connections that are longer than a certain threshold may be indicated in a more prominent manner than shorter connections. In other embodiments, only connection having distances that exceed the threshold may be indicated.

Using the disclosed solution, the physician is provided with a clear real-time visual display that highlights the trajectories and indicate at least the locations where the connection between the ablation points may render its quality insufficient. Using such a display, the physician can revisit the locations in question and complete the ablation procedure successfully.

### SYSTM DESCRIPTION

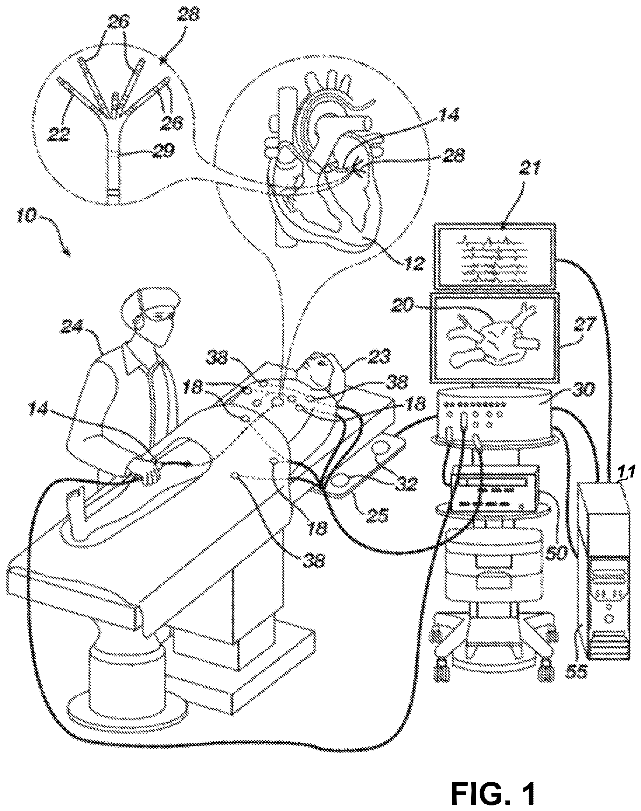

Reference is made to Fig. 1 showing an example catheter-based electrophysiology mapping and ablation system 10. System 10 includes multiple catheters, which may be percutaneously inserted by a physician 24 through the vascular system of a patient 23 into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 may place a distal tip 28 of catheter 14 in contact with the heart wall for sensing a target site in heart 12. For ablation, physician 24 may similarly place a distal end of an ablation catheter in contact with a target site for ablating tissue.

Catheter 14 is an exemplary catheter that includes one and preferably multiple electrodes 26 optionally distributed over a plurality of splines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. The real time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

Additionally or alternatively, system 10 may include one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 may record and display electrograms 21 captured with body surface ECG electrodes 18 and IEGM captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 may be configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software stored therein, and user interface capability. Workstation 55 may provide multiple functions, optionally including (1) modeling the endocardial anatomy in three-dimensions (3D) and rendering the model or anatomical map 20 for display on a display device 27; (2) displaying on display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

In the description below the terms "sites", "ablation sites", "locations", "ablation locations" or the like are used interchangeably and are to be widely construed to indicate a location at which ablation has been applied.

In the description below the terms "connection" and "distance" may be used interchangeably and are to be widely construed to indicate a segment connecting two ablation sites. The term "distance", "distance length" or "distance weight" may be construed, according to the context, as Euclidean distance, distance along a bodily tissue, or any of the above in combination with additional factors, as detailed below.

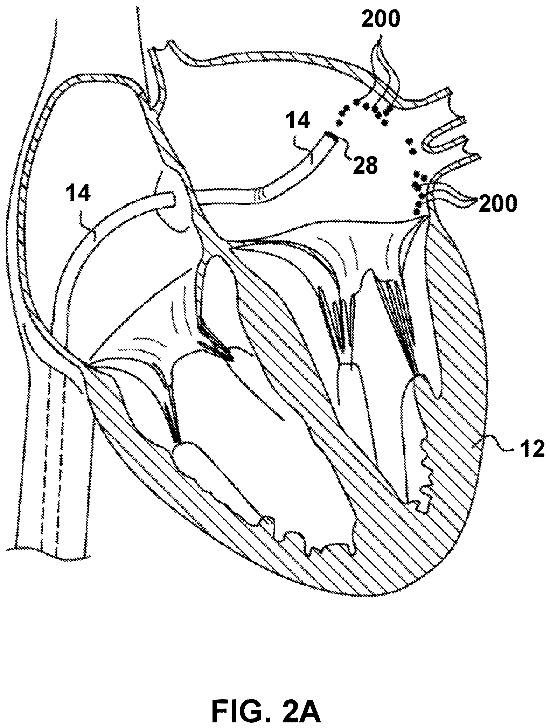

Referring now to Fig. 2A, showing a schematic illustration of heart 12 undergoing ablation, in accordance with an embodiment of the present invention. The ablation electrode at distal end 28 of catheter 14 contacts the heart cavity at multiple ablation sites 200 to induce local necrosis of the heart tissue. A lesion is formed at each ablation site 200.

Using position tracking of a position sensor at distal end 28, or imaging systems as described previously, a recorder such as recorder 11 of Fig. 1 may record the positions of ablation sites 200 as physician 24 forms the multiple lesions on the surface of the heat cavity with the ablation electrode. The position of ablation sites 200 can be displayed to a user such as physician 24 in real time, stored, and presented at a later time after the operation.

Electrophysiology (EP) mapping electrodes near distal end 28 of catheter 14 or of a separate catheter can be used to monitor changes in the electro-cardiac signals measured by the one or more mapping electrodes in response to the ablation therapy, as described previously.

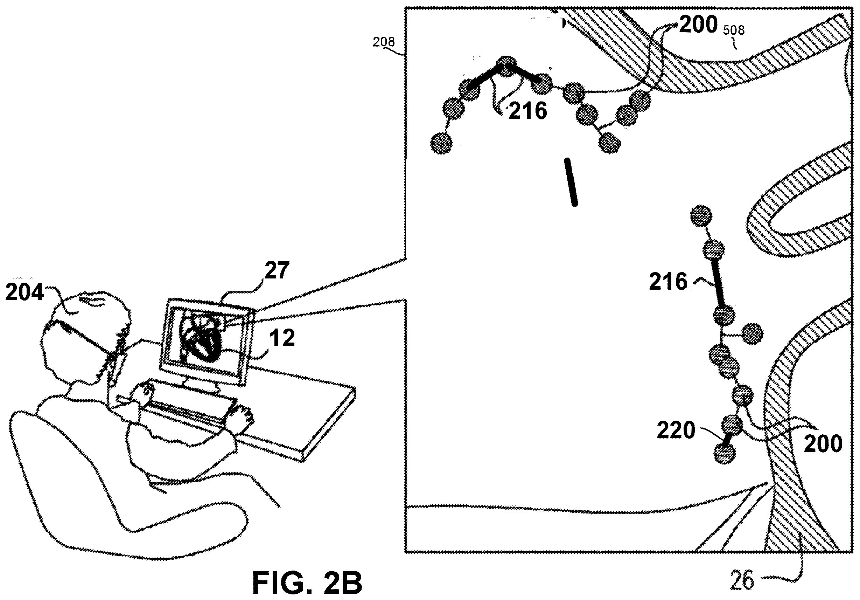

Referring now to Fig. 2B, showing a schematic illustration of a user 204, which may or may not be the same as physician 24, examining an image of heart 12 with annotations related to ablation sites 200, as displayed over display device 27, in accordance with some embodiments of the disclosure.

Fig. 2B shows an enlarged view of the ablation region in Fig. 2A in an inset 208 of ablation sites 200 after processing by a processor, such as a processor of workstation 55.

For some ablation sites 200, the closest ablation site may be at a distance equal to or longer than a threshold away, which may thus be intolerable, since parasitic electrical pathways of the cardiac activation wave may not be eliminated. The threshold may have a default value or may be set by user 204. The threshold may also depend on the specific location within heart 12. The connections having intolerable distances or distance weights may be highlighted for user 204 with bold lines 216.

It is appreciated that some connections may be longer than they visually appear, such as the connection along line 220, which is thus also bold.

The diagrams of Figs. 2A and 2B are depicted merely for conceptual clarity and not by way of limitation of the embodiments of the present invention. Although the ablation locations in Figs. 2A and 2B are shown as open trajectories, the disclosure is equally applicable to showing closed trajectories, as created for example for isolating the pulmonary veins.

It is noted that in Figs. 2A and 2B, for the sake of clarity, lesions at multiple ablation sites 200 are shown to be round, and the diameters of the lesions are shown to be roughly the same. In some situations, one or more ablation sites 200 may not be round but rather amorphic, and the diameters of ablation sites 200 may differ from one another, for example depending on the ablation parameters (e.g., ablation time or ablation signal power) applied at each ablation site. In some embodiments, the distances measured between ablation sites 200 are oblivious to the site diameter. For example, the distances may be computed between the ablation site centers or centers of mass. In other embodiments, the distances measured between ablation sites 200 may depend on the sites' diameters. For example, for the same ablation site center points, the distance between large-diameter lesions may be smaller than the distance between small-diameter lesions.

In some embodiments, a processor of workstation 55 may also scale the distance weight between ablation sites by a scaling factor that depends on the ablation quality (also referred to as ablation index) or other factors of one or both of the ablation sites, as detailed below. As a result, low-quality ablation sites will be interpreted as being further apart from other sites than high-quality sites.

In some applications, such as medical applications and in particular atrial ablation applications, the distances may be calculated over the relevant body tissue rather than as Euclidean distances, which may generally increase the distances.

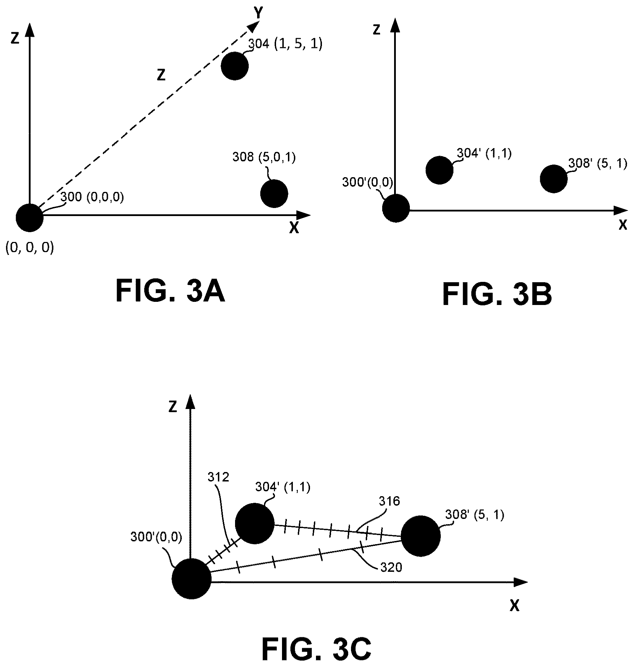

Referring now to Figs. 3A and 3B demonstrating the effect of a larger distance appearing shorter in a projection over a plane, such as the display plane of a visual display device.

Fig. 3A shows three 3D points, point 300 having coordinates (0, 0, 0), point 304 having coordinates (1, 5, 1), and point 308 having coordinates (5, 0, 1). The distances are therefore: 300-304: sqrt(27), 304-308: sqrt(42), 308-300: sqrt (26), i.e., the longest distance is between points 304 and 308 and the shortest is between 300 and 308.

When the points are projected on the X-Z plan as seen in Fig. 3B, and as may be shown on display device 27, the coordinates become 300' (0,0), 304' (1,1) and 308' (5,1), and the corresponding distances are thus 300'-304': sqrt(2), 304'-308': sqrt(16), 308'-300': sqrt (26). Thus, the longer distance appears to be between points 300' and 308' and the shortest is between 300' and 304', which does not reflect the relations between the actual 3D distances.

It may thus be particularly important to highlight the distances that exceed the threshold, since they may not be perceived as such by a user, such as a physician examining an image of heart 12, and may therefore lead to sub optimal ablation results.

Referring now to Fig. 3C, showing the points as in Fig. 3B, with supplemented connecting lines, in accordance with some embodiments of the disclosure.

The connecting lines between points, wherein the points may represent ablation sites, are supplemented with tick marks, wherein the distance between any two consecutive tick marks is constant for the whole image. Thus, each of segment 312 connecting points 300' and 304' and segment 320 connecting points 300' and 308' comprises four tick marks, as the distances are almost the same length (sqrt(26) and sqrt(27)), such that rounding the distances ends up in the same number of tick marks. Segment 316 connecting 304' and 308', whose length is sqrt(42) has seven (7) tick marks, thus it is understood that it represents a longer distance than the other segments, although in the projection it appears shorter than segment 320.

As noted above, the distances may be calculated as Euclidean distances or over the relevant body tissue. Thus, since the distance may be even longer than the Euclidean distance, the number of tick marks may even increase.

It is appreciated that the length of a connecting segment, also referred to as connection, may additionally or alternatively be represented by a color, a shade, a pattern or another characteristic of the segments. For example, longer connections may be indicated by a darker shade, a denser pattern, or the like. However, the tick marks also provide a quantitative measure of the connection, which lets the user easily assess the distance. It is also appreciated that two or more indication methods may be used simultaneously, such as tick marks and/or pattern and/or color and/or shade and/or width.

In some embodiments, all connecting segments may be displayed such that the physician may see the full ablation trajectory, wherein connections having a higher weight may be displayed in a more prominent manner. In other embodiments, the segments may be displayed and supplemented only if the distance between an ablation site and the closest ablation site exceeds a threshold, such that the physician may easily detect areas in which more ablation sites may be required, without unnecessarily cluttering the image.

In further embodiments, connections between ablation sites may also be displayed, for example in Augmented Wide Area Circumferential Catheter (WACA) ablation cases where the ablation surrounds two pulmonary veins.

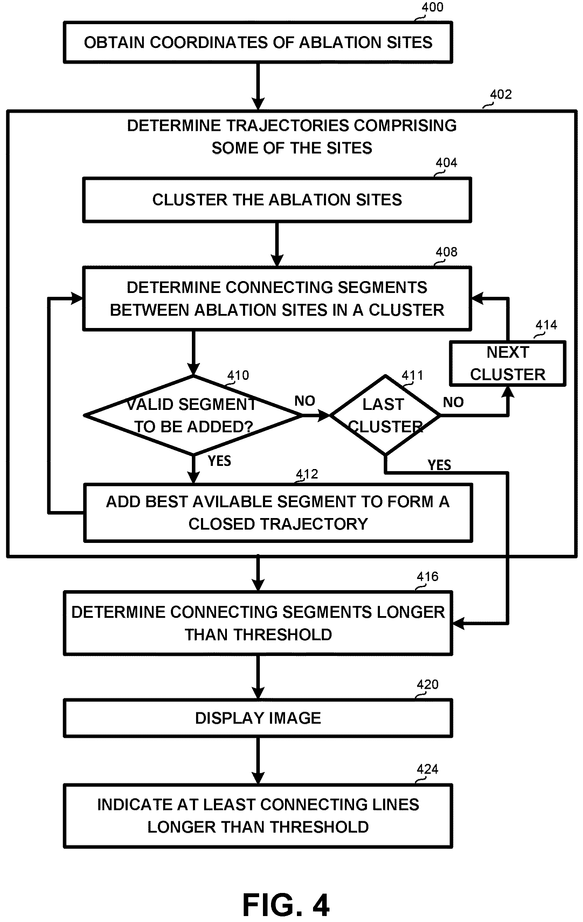

Referring now to Fig. 4, showing a flowchart of steps in a method for determining and displaying trajectories connecting ablation sites, and connections therebetween, in accordance with some embodiments of the disclosure.

On step 400, coordinates of ablation sites may be obtained. The data may be obtained from an ablation system during an ablation operation, retrieved from a storage device where they have been stored during or after an ablation operation, or the like. The ablation sites may be obtained during the operation when the physician checks whether additional sites need to be ablated, or at a later time when it is required to examine the ablation results.

The ablation sites may be received as 3D points, optionally with additional parameters such as an ablation index indicating the ablation quality, a time stamp at which the ablation was created, or the like.

The ablation sites may be regarded as nodes in a graph, and a virtual segment connecting two ablation sites may be interpreted as an edge in the graph. An edge may be unidirectional or bidirectional, wherein in the current disclosure, the edges may be bidirectional, i.e., not associated with a particular direction.

Each edge may have a weight, wherein the weight may be equivalent to the length of the segment.

It is appreciated that a circle in a graph relates to a closed trajectory connecting a plurality of nodes, and not necessarily to a geometrical circle.

On step 402, one or more trajectories, each comprising at least some of the locations may be determined, wherein each such trajectory indicates an ablation operation designed to electrically isolate an area of the heart. It is appreciated that ablation trajectories may be open as in roof ablation, or closed as in circumferential ablation.

Step 402 may include step 404, in which the ablation sites may be clustered. Clustering may generally refer to dividing nodes within a graph into groups wherein the distances between nodes within the same group are smaller than the distances between nodes assigned to different clusters. Thus, the ablation sites may be regarded as nodes in a graph, and a connection between two ablation sites is an edge. Each cluster may thus represent the ablation sites performed for electrically isolating a specific area of the heart, such as a one or a pair of pulmonary veins.

Clustering may be performed using any currently known method, or any method that will become known in the future, such as but not limited to K-means clustering, Mean-Shift Clustering, Density-Based Spatial Clustering of Applications with Noise (DBSCAN), Expectation-Maximization (EM) Clustering using Gaussian Mixture Models (GMM), Affinity Propagation, Agglomerative Clustering, BIRCH, Mini-Batch K-Means, OPTICS, Spectral Clustering, or others.

In some embodiments, multiple clustering methods may be applied, and the clustering to be used may be selected by majority voting.

Clustering may use the distance between sites as a connection weight wherein the distance between two ablation points may be calculated as the Euclidean distance, but may also be calculated as the distance along the cardiac tissue. Additionally or alternatively, the connection weight may take into account additional factors. For example, the distance between ablation sites may be scaled by a scaling factor that also integrates the size of an alation site, the quality (also referred to as ablation index) of one or both of the ablation sites. As a result, low-quality ablation sites may be interpreted as being further apart from other sites than high-quality sites. Additional factors may include the temperature with which the lesion was created at ab ablation location, one or more anatomy factors such as the heart wall thickness at the area of the lesion, or the like.

Step 402 may include step 408, in which connecting segments, also referred to as edges, may be determined between the ablation sites within each cluster.

In some embodiments, the connecting segments may form a spanning tree, and in particular a minimal spanning tree. In graphs, a minimal spanning tree comprises all the nodes of the graph, and a number of edges which is equal to the number of nodes minus one, such that no cycles are formed. A minimal spanning tree has the minimal total edge weight, wherein the weight of each edge is as detailed above, and the total edge weight is the sum of the weights of all edges in the tree. In some embodiments, one or more locations within a cluster may be left out and not be part of the spanning tree, for example if the total weight without this location is significantly lower than with this location.

The minimal spanning tree can be found using any currently known algorithm, or an algorithm that will become known in the future, such as but not limited to Boruvka's algorithm, Prim's algorithm, Kruskal's algorithm or others.

On step 410, it may be determined whether additional segments may be added, which form a closed trajectory when added to the spanning tree. It is appreciated that in further iterations the segments may be added on top of the spanning tree and the segments added on previous iterations. However, an edge that closes a trajectory may not always comply with predetermined conditions and may thus be eliminated from being added, for example in roof ablations, wherein an edge connecting the first and last location sites may be too long.

In some examples, step 410 may comprise activating an algorithm, such as the Floyd-Warshall algorithm, that computes for each pair of nodes within the current graph the total weight of a path between the nodes, wherein the path is comprised of edges existing in the spanning tree or previously added. It is appreciated that since the graph is a tree, then before an edge has been added, there is a single path between any two nodes. For each such pair of nodes, the distance, such as the Euclidean distance is calculated as well.

It may then be determined whether a pair of nodes for which the tradeoff between the path weight and the distance is optimal, also complies with one or more predetermined conditions, as detailed below. The tradeoff may be aimed at selecting an edge having a minimal weight, where the total weight within the graph of the path connecting the edge nodes is maximal. Intuitively speaking, the algorithm aims at finding the shortest edge that creates the largest circuit.

It is appreciated that the weight calculation as detailed above may be applicable for calculating the weight of an edge when determining the minimal spanning tree and the path weight, as well as for calculating the connection weight between two ablation locations not yet connected by an edge.

If such segment exists ("YES"), then on step 412 the segment may be added to the graph, and form a closed trajectory. Execution may them return to step 410 to search for additional segments.

It is appreciated that the algorithm is non-trivial, as any three or more nodes and the path connecting them can be closed into a loop. However, connecting very few points is likely to find a local circuit which does not cover a closed trajectory of ablation locations, while connecting too many points may introduce a noisy picture.

In some situations, for example in roof ablation, the edge between the nodes that provides the best tradeoff may not comply with a predetermined condition, for example its weight, Euclidean distance or distance along the body tissue may exceed a predetermined threshold. Again, intuitively speaking, this may be the case where the ablation locations form an elongated shape and are not intended to form a circuit.

Thus, if there is no segment to be added, or a maximal number of edge-adding iterations has been performed, for example 5-20 iterations such as 10 iterations, execution may continue on step 414 in selecting the next cluster and proceeding as detailed above for the next cluster. If no other cluster exists as determined on step 411, execution may continue to step 416 as detailed below.

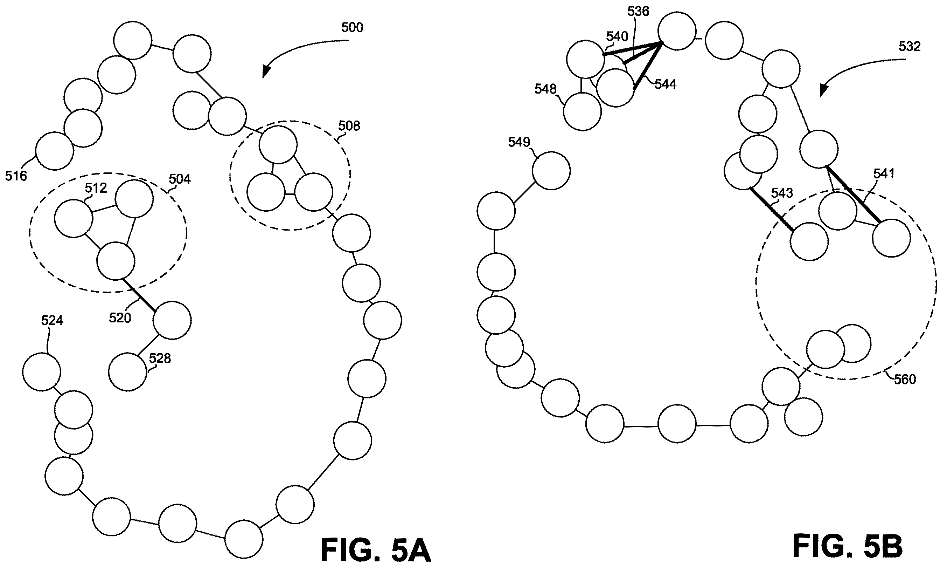

Referring now to Figs. 5A and 5B, showing two examples of ablation locations and connections therebetween as created by a traditional solution. Fig. 5A shows graph 500 that comprises two local circuits 504 and 508, which may be confusing to a physician as they are two small to represent a closed trajectory. In addition, graph 500 shows no connection between locations 512 and 516, and between locations 524 and 528.

Similarly, graph 532 of Fig. 5B demonstrates disconnection between locations 548 and 549, and another disconnection as shown in area 560. Additionally, segment 541 unnecessarily closes an unrequired circuit, which may be further confusing.

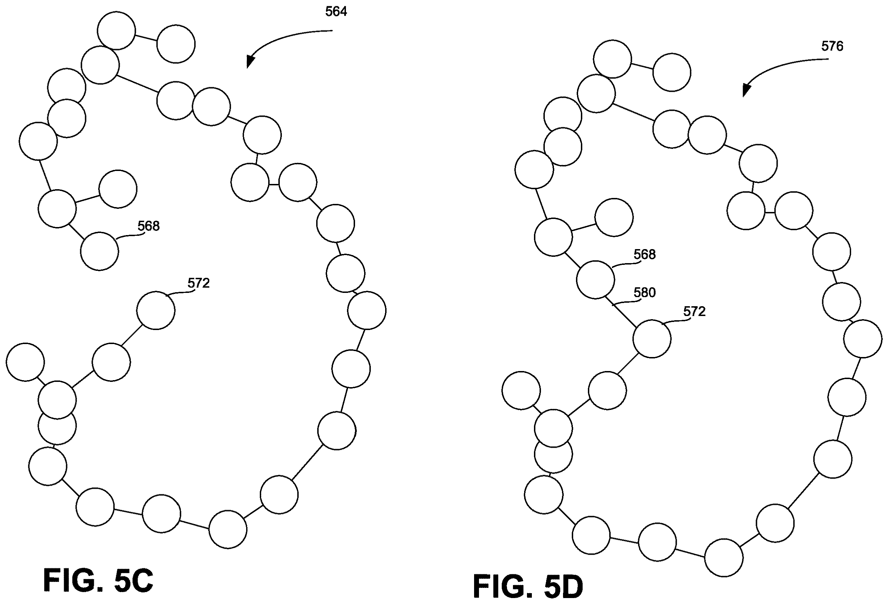

Referring now to Figs. 5C-5F, showing examples of calculating connections, in accordance with some exemplary embodiments of the disclosure. It is appreciated that the examples of Figs. 5C-5F take into account only the Euclidean distance between points as the weight of a connection. Some locations in any of Figs. 5C-5F seem to partly or fully overlap or touch each other, such locations are considered to be pairwise connected to each other.

Fig. 5C shows graph 564 having the same node locations as Fig. 5A above, after calculating the minimal spanning tree. It is seen that the graph, being a tree, contains no closed circuits, and thus eliminates the local circuits of graph 500. Additionally, graph 564 does not have any disconnected nodes or groups of nodes.

Fig. 5D shows graph 576 having the same node locations as in Fig. 5C, after operating the Floyd-Warshall algorithm, and selecting edge 580 for forming a closed trajectory. The result is a large circuit, with no discontinuities and with no local loops.

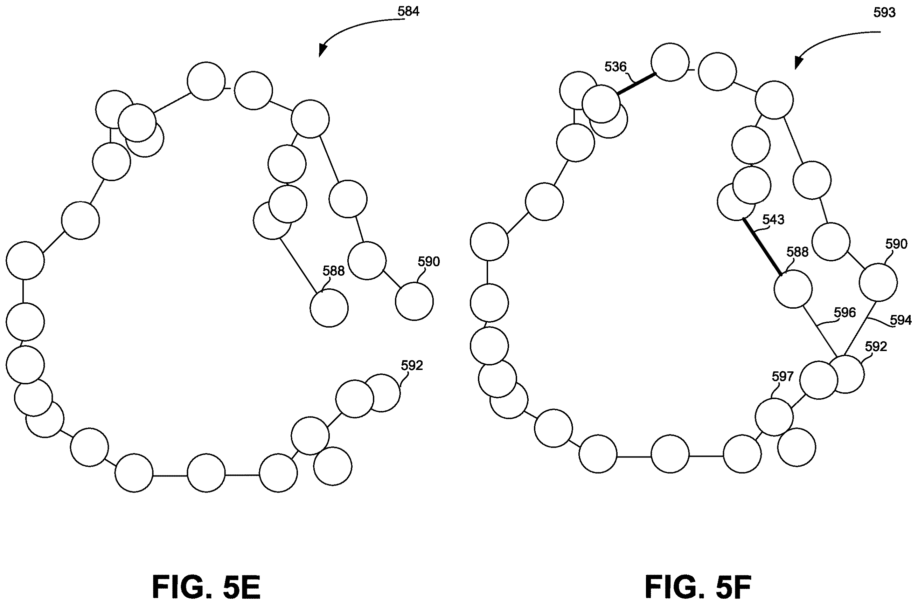

Fig. 5E shows graph 584 having the same ablation locations as in Fig. 5B above, after calculating the minimal spanning tree. It is seen that the graph, being a tree, contains no closed circuits, and thus eliminates the local circuits of graph 500. Additionally, graph 584 does not have any disconnected nodes or groups of nodes.

Fig. 5F shows graph 593 having the same ablation locations as in Fig. 5E, after operating the Floyd-Warshall algorithm and selecting edges to be added as described above. Thus, edges 594 and 596 have been added which close two circuits, one containing both locations 588 and 590, and the other containing either of 588 or 590, and node 597. The result is a large circuit, with no discontinuities and no local loops.

When determining the edge to be added, some morphological considerations may be taken into account. For example, the roundness of the circle may be considered, for example by estimating the standard deviation of the locations from the center of mass of the created shape, and preferring circuits having low standard deviation.

In some exemplary embodiments, the presence or absence of one or more edges connecting ablation sites may be subject to the user's consideration. Thus, a user may mark certain edges, or certain pairs of ablation sites as being connected, and/or mark other edges or pairs of ablation sites as not having a connection. These indications may be used as constraints in the creation of the trajectories, i.e., the presence of an edge will be forced or eliminated where so indicated by the user.

On step 416, it may be determined which segments that connect ablation sites are longer than a threshold value, such as between 3mm and 10mm, for example 4mm, meaning that the ablation points connected by the segment may be too far away from each other to ensure electrical isolation. It is noted that this threshold may be different, for example shorter, than the threshold used as a condition on step 410 for determining connecting edges to be added. For example, if on step 410 no segment longer than 6mm is added, the threshold used on step 416 may be applicable to the added segments, and may indicate segments longer than 4mm. It is seen that while the traditional method indicated a plurality of long segments, such as segment 520 of Fig. 5A and segments 536, 540, 544, 541 and 543 of Fig. 5B, wherein some of which are clearly unnecessary such as 540 and 541, the current disclosure only indicated segments 536 and 543 as being too long, as seen in Fig. 5F.

On step 420, an image of the heart or part thereof may be displayed, for example as image 20 on display device 27, or as shown in image 208.

On step 424, at least segments which are longer than the threshold, as identified on step 416, may be indicated on the image. However, the other segments forming the closed trajectories may be indicated as well. Additionally, the length of the displayed segments may be visually indicated as detailed above, by tick marks, pattern, color, shade, width, or the like, or a combination of two or more of the above. In other embodiments, all segments may be indicated on the display, and

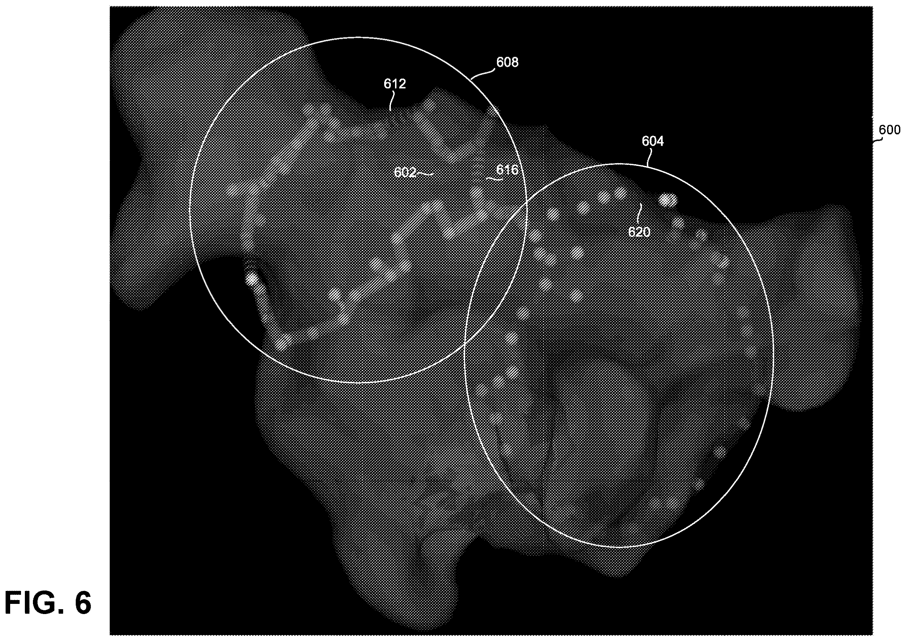

Referring now to Fig. 6, showing an image 600 of a heart as displayed over a display device, with the ablation points, and with two estimated trajectories 604 and 608, in accordance with some embodiments of the disclosure.

It is appreciated that some ablation points, such as 602, are not part of any such circle.

It is also appreciated that some segments, such as 612, 616, 620 are shown with marks, indicating that they may be longer or having higher weight than a threshold (although this may not seem that way, due to the 2D projection), and should be further examined.

Different circuits, such as 604 and 608, may or may not be displayed in different color schemes, in order to better visually differentiate them.

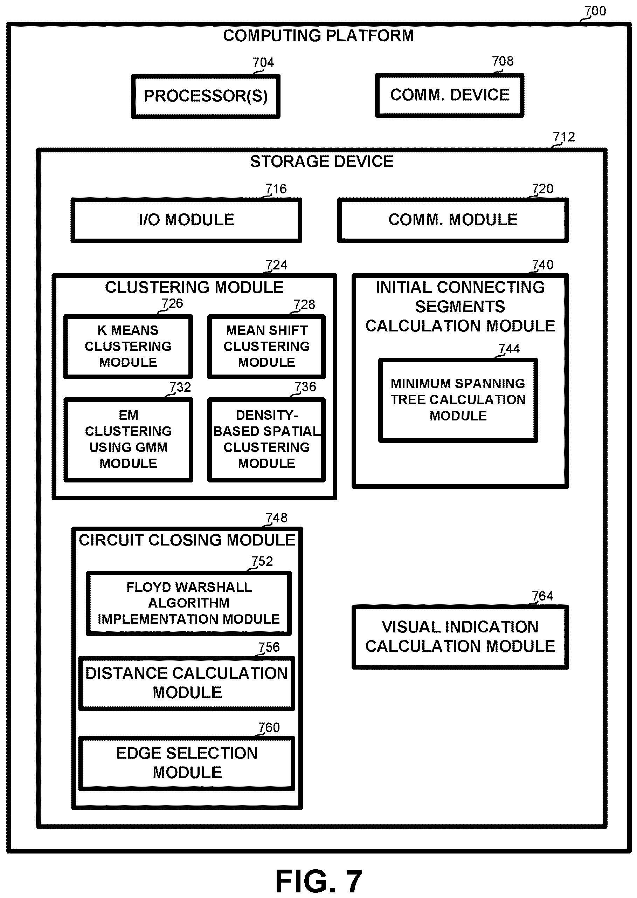

Referring now to Fig. 7, showing a block diagram of a computing platform 700 for determining whether a connection between ablation points exceeds a predetermined threshold, in accordance with some exemplary embodiments of the disclosure.

It will be appreciated that computing platform 700 may be embedded within workstation 55, but may also be a standalone computing platform or embedded elsewhere and be in operative communication with workstation 55.

Computing platform 700 may be implemented as one or more computing platforms which may be operatively connected to each other. For example, one or more remote computing platforms, which may be implemented for example on a cloud computer. Other computing platforms may be a part of a computer network of the associated organization. In other embodiments, all the functionality may be provided by one or more computing platforms all being a part of the organization network.

Computing platform 700 may comprise one or more processors 704 located on the same computing platform or not, which may be one or more Central Processing Units (CPU), microprocessors, electronic circuits, Integrated Circuits (IC) or the like. Processor 704 may be configured to provide the required functionality, for example by loading to memory and activating the software modules stored on storage device 712 detailed below.

Computing platform 700 may comprise a communication device 708 for communicating with other devices or other computing platforms, for example obtaining information from the catheterization controller, storing and retrieving data to or from remote storage devices, or the like. Communication module 708 may be adapted to interface with any communication channel such as Local Area Network (LAN), Wide Area Network (WAN), cellular network or the like, and use any relevant communication protocol.

Computing platform 700 may comprise a storage device 712, such as a hard disk drive, a Flash disk, a Random Access Memory (RAM), a memory chip, or the like. In some exemplary embodiments, storage device 712 may retain program code operative to cause processor 704 to perform acts associated with any of the modules listed below, or steps of the method of Fig. 4 above. The program code may comprise one or more executable units, such as functions, libraries, standalone programs or the like, adapted to execute instructions as detailed below.

Alternatively or additionally, the provided instructions may be stored on non-transitory tangible computer-readable media, such as magnetic, optical, or electronic memory.

Storage device 712 may comprise I/O module 716, for rendering a display to the user to be displayed over display device 27, such as a map of the heart, ablation points and indications of distances therebetween, or the like. I/O module 716 may also be operative in receiving instructions and operation parameters from the user.

Storage device 712 may comprise communication module 720 for transmitting and receiving data to and from other systems, such as the ablation system, external storage devices, or the like.

Storage device 712 may comprise clustering module 724, which may comprise one or more modules for clustering sites, such as but not limited to K-means clustering module 726, mean shift clustering module 728, EM clustering using GMM module 732, density-based clustering module 736, or others.

Storage device 712 may comprise initial connecting segments calculation module 740, which may comprise one or more modules for calculating segments connecting ablation sites, such as but not limited to minimum spanning tree calculation module 744 (which may be implemented in a plurality of ways by a plurality of algorithms), or others.

Storage device 712 may comprise circuit closing module 748, which may comprise one or more modules for determining how to close a spanning tree into a closed trajectory.

Circuit closing module 748 may comprise a module for calculating the weights of paths between any two nodes within a tree, such as but not limited to Floyd-Warshall algorithm implementation module 752, or others.

Circuit closing module 748 may comprise distance calculation module 756, for calculating the distances or weights between nodes representing ablation sites, and/or determining whether one or more of the edge weights exceeds the threshold.

Circuit closing module 748 may comprise edge selection module 760 for selecting edges to be added to the initial segments, for example based on a tradeoff between the weight of a path connecting two locations as calculated by Floyd-Warshall algorithm implementation module 752 (or another algorithm) and the distance between the locations as calculated by distance calculation module 756. Circuit closing module 748 may also determine that no edge is to be added, such that one or more trajectories are not closed.

Storage device 712 may comprise visual indication calculation module 764, for determining how to visually display the connections. In some embodiments, all connecting segments may be indicated in an image, and the connecting segment whose length exceeds the threshold may be marked accordingly. In other embodiments, only the connecting segment whose length exceeds the threshold may be marked. The marking may take various forms, in particular adding tick marks at constant distances, wherein the distance between two tick marks indicates a constant distance or a constant weight in reality, such that the number of tick marks along a segment indicates its length. Alternative or additional marking methods may also be used, such as pattern, width, color, shade, or the like.

It is appreciated that the steps and modules disclosed above are in addition to the software, hardware, firmware or other modules required for operating a catheter, displaying the catheterization process, generating the heart map, or the like. Further details for methods and systems may be found, for example in US8676305, US9629567, incorporated herein by reference in their entirety for any purpose.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture instructions, machine instructions, machine dependent instructions, microcode, firmware instructions, state-setting data, or either source code or object code written in any combination of one or more programming languages, programming languages such as Java, C, C*++,* Python, or others. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

### EXAMPLES

### Example 1

A method comprising: obtaining coordinates of locations in a body part of a patient; determining at least one trajectory comprising at least part of the locations; determining whether a connection between at least two locations connected along the at least one trajectory exceeds a threshold; providing a visual representation of the body part, including at least some of the locations and visual indications of connections between locations; and subject to the connection exceeding the threshold, providing a visual indication of the connection weight.

### Example 2

The method according to example 1, wherein determining the at least one trajectory comprises: clustering the locations to form at least one cluster; and determining a tree within each cluster.

### Example 3

The method according to example 2, wherein the tree is a minimal spanning tree.

### Example 4

The method according to example 2, further comprising: determining at least one segment connecting locations within the cluster, and adding the segment to the tree such that the tree forms at least one closed trajectory.

### Example 5

The method according to example 4, wherein determining the at least one segment that forms at least one closed trajectory uses the Floyd-Warshall Algorithm.

### Example 6

The method according to example 4, wherein determining the at least one segment that forms at least one closed trajectory uses a tradeoff between a weight of a path connecting two locations within a tree and a distance between the two locations.

### Example 7

The method according to example 1, wherein the locations are locations of ablation sites within a body of the patient.

### Example 8

The method according to example 1, wherein the at least one trajectory encircles two pulmonary veins of the patient.

### Example 9

The method according to example 1, wherein the visual indication comprises tick marks, and wherein a number of tick marks along a segment connecting the at least two locations is indicative of a length of the segment.

### Example 10

The method according to example 1, wherein the connection is quantified as a Euclidean distance.

### Example 11

The method according to example 1, wherein the connection is quantified as a distance along a tissue of the patient.

### Example 12

The method according to example 1, wherein the at least one trajectory is at least one closed trajectory.

### Example 13

The method according to example 1, wherein determining the at least one trajectory or the distance uses also at least one factor for assessing a connection, the at least one factor selected from the group consisting of: an ablation size, an ablation index, temperature at which a location has been ablated, and an anatomy feature.

### Example 14

The method according to example 13, wherein the anatomy feature is a thickness of heart wall in a lesion location.

### Example 15

The method according to example 1, wherein the at least one trajectory comprises at least one segment indicated by the user as existing.

### Example 16

The method according to example 1, wherein the at least one trajectory does not comprise at least one segment indicated by the user as absent.

### Example 17

A computerized apparatus having a processor coupled with a memory unit, the processor being adapted to perform the steps of: obtaining coordinates of locations in a body part of a patient; determining at least one trajectory comprising at least part of the locations; determining whether a connection between at least two locations connected along the trajectory exceeds a threshold; providing a visual representation of the body part, including at least some of the locations and visual indications of connections between locations; and subject to the connection exceeding the threshold, providing a visual indication of the connection weight.

### Example 18

The computerized apparatus according to example 17, wherein the processor is adapted to perform the following steps for determining the at least one trajectory comprises: clustering the locations to form at least one cluster; and determining a minimal spanning tree within each cluster.

### Example 19

The computerized apparatus according to example 18, wherein the processor is adapted to determine at least one segment connecting locations within the cluster, and to add the segment to the tree such that the tree forms at least one closed trajectory.

### Example 20

A computer program product comprising a non-transitory computer readable medium retaining program instructions, which instructions when read by a processor, cause the processor to perform: obtaining coordinates of locations in a body part of a patient; determining at least one trajectory comprising at least part of the locations; determining whether a connection between at least two locations connected along the trajectory exceeds a threshold; providing a visual representation of the body part, including at least some of the locations and visual indications of connections between locations; and subject to the connection exceeding the threshold, providing a visual indication of the connection weight.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method comprising:
obtaining coordinates of locations in a body part of a patient;
determining at least one trajectory comprising at least part of the locations;
determining whether a connection between at least two locations connected along the at least one trajectory exceeds a threshold;
providing a visual representation of the body part, including at least some of the locations and visual indications of connections between locations; and
subject to the connection exceeding the threshold, providing a visual indication of the connection weight, wherein the visual indication comprises tick marks, and wherein a number of tick marks along a segment connecting the at least two locations is indicative of a length of the segment.

2. The method of Claim 1, wherein determining the at least one trajectory comprises:
clustering the locations to form at least one cluster; and
determining a tree within each cluster.

3. The method of Claim 2, wherein the tree is a minimal spanning tree.

4. The method of Claim 2, further comprising:
determining at least one segment connecting locations within the cluster; and
adding the segment to the tree such that the tree forms at least one closed trajectory.

5. The method of Claim 4, wherein determining the at least one segment that forms at least one closed trajectory uses the Floyd-Warshall Algorithm.

6. The method of Claim 4, wherein determining the at least one segment that forms at least one closed trajectory uses a tradeoff between a weight of a path connecting two locations within a tree and a distance between the two locations.

7. The method of Claim 1, wherein the locations are locations of ablation sites within a body of the patient.

8. The method of Claim 1, wherein the at least one trajectory encircles two pulmonary veins of the patient.

9. The method of Claim 1, wherein the connection is quantified as a Euclidean distance.

10. The method of Claim 1, wherein the connection is quantified as a distance along a tissue of the patient.

11. The method of Claim 1, wherein the at least one trajectory is a closed trajectory.

12. The method of Claim 1, wherein determining the at least one trajectory or the distance uses also at least one factor for assessing a connection, the at least one factor selected from the group consisting of: an ablation size, an ablation index, temperature at which a location has been ablated, and an anatomy feature.

13. The method of Claim 12, wherein the anatomy feature is a thickness of heart wall in a lesion location.

14. The method of Claim 1, wherein the at least one trajectory comprises at least one segment indicated by the user as existing.

15. The method of Claim 1, wherein the at least one trajectory does not comprise at least one segment indicated by the user as absent.

16. A computerized apparatus having a processor coupled with a memory unit, the processor being adapted to perform the steps of:
obtaining coordinates of locations in a body part of a patient;
determining at least one trajectory comprising at least part of the locations;
determining whether a connection between at least two locations connected along the at least one trajectory exceeds a threshold;
providing a visual representation of the body part, including at least some of the locations and visual indications of connections between locations; and
subject to the connection exceeding the threshold, providing a visual indication of the connection weight, wherein the visual indication comprises tick marks, and wherein a number of tick marks along a segment connecting the at least two locations is indicative of a length of the segment.

17. The computerized apparatus of Claim 16, wherein the processor is adapted to perform the following steps for determining the at least one trajectory comprises:
clustering the locations to form at least one cluster; and
determining a minimal spanning tree within each cluster.

18. The computerized apparatus of Claim 17, wherein the processor is adapted to determine at least one segment connecting locations within the cluster, and to add the segment to the tree such that the tree forms at least one closed trajectory.

19. A computer program product comprising a non-transitory computer readable medium retaining program instructions, which instructions when read by a processor, cause the processor to perform:
obtaining coordinates of locations in a body part of a patient;
determining at least one trajectory comprising at least part of the locations;
determining whether a connection between at least two locations connected along the at least one trajectory exceeds a threshold;
providing a visual representation of the body part, including at least some of the locations and visual indications of connections between locations; and
subject to the connection exceeding the threshold, providing a visual indication of the connection weight, wherein the visual indication comprises tick marks, and wherein a number of tick marks along a segment connecting the at least two locations is indicative of a length of the segment.
